(19) Europäisches Patentamt
European Patent Office
Office européen des brevets

(11) Publication number: **0 202 911 B1**

# (12) EUROPEAN PATENT SPECIFICATION

(45) Date of publication of patent specification: **14.03.90**

(51) Int. Cl.⁵: **B 01 J 27/22**, B 01 J 37/34, C 07 C 1/04, C 07 C 9/14, C 07 C 11/02

(21) Application number: **86303811.3**

(22) Date of filing: **20.05.86**

## (54) Iron-carbon-based catalysts and a method for the production of hydrocarbons using those catalysts.

(30) Priority: **20.05.85 US 735768**
**20.05.85 US 735769**

(43) Date of publication of application:
**26.11.86 Bulletin 86/48**

(45) Publication of the grant of the patent:
**14.03.90 Bulletin 90/11**

(84) Designated Contracting States:
**DE GB NL**

(56) References cited:
**EP-A-0 131 465**
**US-A-4 134 907**
**US-A-4 468 474**
**US-A-4 472 513**

**J.R. Anderson and M. Boudart "Catalysis", vol. 1, 1981 SPRINGER-VERLAG, Berlin**

(73) Proprietor: **Exxon Research and Engineering Company**
**P.O.Box 390 180 Park Avenue**
**Florham Park New Jersey 07932 (US)**

(72) Inventor: **Fiato, Rocco Anthony**
**275 Country Club Lane**
**Scotch Plains New Jersey 07076 (US)**
Inventor: **Rice, Gary Wayne**
**P.O. BOX 360DD, RD2**
**Whitehouse Station New Jersey 08889 (US)**
Inventor: **Soled, Stuart Leon**
**RD1, P.O BOX 117 Cooks Cross Road**
**Pittstown New Jersey 08867 (US)**

(74) Representative: **Somers, Harold Arnold et al**
**ESSO Engineering (Europe) Ltd. Patents & Licences Apex Tower High Street**
**New Malden Surrey KT3 4DJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a finely divided or supported iron carbide-based catalyst and in particular a catalyst which is produced by a gas phase pyrolytic decomposition reaction driven by a laser and the use of such a catalyst to produce various hydrocarbons, typically paraffin waxes or olefins, from CO and $H_2$.

The Fischer-Tropsch reaction involves the catalytic hydrogenation of carbon monoxide to produce a variety of products ranging in size and functionality from methane to higher alcohols. The methanation reaction was first described by Sabatier and Senderens in 1902. The later work of Fischer and Tropsch dealing with higher hydrocarbons was described in Brennstoff-Chem. *7*, 97 (1926).

The reaction is highly exothermic and care must be taken to design reactors for adequate heat exchange capacity. Nevertheless, substantial research has been undertaken in the interim since the initial characterization of the reaction during the 1920's. The process is especially suitable for use when carbonaceous feedstocks of otherwise low economic value are available. For instance, the first major commercial use of the Fischer-Tropsch process was in Germany during the mid-30's. By the beginning of World War II, Germany was producing nearly 11,000 B/D (1747900 liter/day) of primary products using mainly the cobalt-based catalyst described by Fischer and Pichler (DE—A—731,295—issued August 2, 1936). The feedstock was, in general, based on available coals.

Subsequently, a consortium of nine American companies designed and built a plant at Brownsville, Texas based on an iron-based catalyst. The plant was completed in 1950 and had a design capacity of 50MMSCFD (1416 MM liter/day). Various economic and technical difficulties caused final shutdown of the plant in the late 50's.

A reasonably economic use of the process has been practised in South Africa in the Sasol plants. These plants use an iron-based catalyst and produce gasoline and waxes by gasifying a somewhat low-grade coal to produce a synthesis gas for feed to the Fischer-Tropsch reactors.

Research continues in this area because of the potential for converting low value feedstocks into higher value products.

The chemistry of the Fischer-Tropsch reactions is, in a gross sense, quite simple. The overall reactions for the production of alkanes (No. 1), alkenes (No. 2) and alcohols (No. 3) are as follows:

$$1.\ \begin{cases} (2n+1)H_2+nCO \rightarrow C_nH_{2n+2}+nH_2O \\ (n+1)H_2+2nCO \rightarrow C_nH_{2n+2}+nCO_2 \end{cases}$$

$$2.\ \begin{cases} 2nH_2+nCO \rightarrow C_nH_{2n}+nH_2O \\ nH_2+2nCO \rightarrow C_nH_{2n}+nCO_2 \end{cases}$$

$$3.\ \begin{cases} 2nH_2+nCO \rightarrow C_nH_{2n+1}OH+(n-1)H_2O \\ (n+1)H_2+(2n-1)CO \rightarrow C_nH_{2n+1}OH+(n-1)CO_2 \end{cases}$$

The types and amount of products obtained via such reactions are typically dependent upon the reaction conditions and choice of catalyst.

Few of the catalysts used in the past have been either very selective or very active. Those catalysts that were selective or active were uneconomic for other reasons, e.g., sensitivity to sulfur poisoning or used high cost catalytic metals such as ruthenium.

The catalyst of the present invention is iron/carbon-based. Because of the method of its preparation, the catalyst has high selectivity and/or conversion at reaction conditions considered to be quite moderate.

As noted above in the historical discussion, iron-bearing catalysts were among the first ever used in the Fischer-Tropsch reaction. Indeed, Fischer and Tropsch believed that carbides were an intermediate in the overall reaction. Later kinetics work suggested carbides could not be an intermediate in the process. Hall *et al*, J. Soc. Chem. Ind. London *65*, 128 (1946); Weller, J. Am. Chem. Soc. *69*, 2432 (1947) and; Kummer *et al*, J. Am. Chem. Soc. *70*, 3632 (1948). However, the reduced metallic iron, as used in the Lurgi-Ruhrchemie fixed bed process appears to change from the original α-Fe phase to a mixture of α-Fe, $Fe_3O_4$, FeC and $Fe_2C$ as conversion operations continue. *See*, Malan *et al* Brennstoff-Chem. *42*, 209—212 (1961).

The present invention, as will be discussed below in greater detail, involves the use of a laser to pyrolize low valence iron-carbon bearing compounds to produce a fine particle iron-carbon containing catalyst. At least a portion of the catalyst is the iron carbide, cementite.

Others have described the use of iron-carbon containing catalysts produced by laser pyrolysis in Fischer-Tropsch reactions. The work of Gupta *et al* (in US—A—4468474), issued August 28, 1984 and in Spie *458*, Appl. of Lasers to Industrial Chemistry, 131—139 (1984) shows the production of iron, carbon and silicon-containing catalysts by a laser and the catalysts' subsequent use in the Fischer-Tropsch process. Moderate activity and high $C_2$—$C_4$ olefin selectivity is asserted for the catalysts.

The catalysts of this invention contain substantially no silicon.

US—A—4134907 describes and claims a process for converting a first gas mixture containing carbon monoxide and hydrogen into a second gas mixture having a substantially increased fuel value, comprising methane, in a single reaction zone which comprises:

(a) maintaining iron and $Fe_3C$ in a fluid bed;

(b) continuouly introducing said first gas mixture into said fluid bed;

(c) maintaining a temperature of about 600°F—1200°F (315.6 to 648.9°C) and a pressure of about 1—10 atmospheres in said fluid bed so that some of the carbon monoxide is reduced to carbon, the iron is reacted with carbon to form $Fe_3C$, and the $Fe_3C$ is reacted with hydrogen to form methane and reform iron; and

(d) continuously removing from said fluid bed as a product said second gas mixture of methane, carbon monoxide and hydrogen having an increased fuel value.

No known prior art is believed to show the use of the catalyst described below in the efficient production of heavy hydrocarbons.

The present invention provides a catalyst composition comprising finely divided non-pyrophoric particles containing iron and carbon components, at least a portion of which is cementite, produced in a reaction zone from a volatile compound containing iron and carbon in the presence of laser radiation under such conditions of laser flux density, power absorption, a volatile iron and carbon compound concentration and pressure sufficient to produce said particles, the particles having average diameters between 1 and 100 nm, and the composition being substantially free of silicon.

The invention also provides a process for producing $C_5$ hydrocarbons from a CO and $H_2$ mixture comprising the step of contacting said mixture at a temperature between 200°C and 250°C with a substantially silicon-free catalyst composition as herein described comprising non-pyrophoric particles of an iron-carbon component, at least a portion of which is cementite and wherein the particles have average diameters between 1 and 100 nm produced by a laser-driven gas phase pyrolytic decomposition of a volatile compound containing iron and carbon, and a promoter selected from alkali metals and alkaline earth metals and preferably present in an amount of from 2 to 10 weight percent based on the catalyst composition.

The invention further provides a process for producing $C_2$—$C_{15}$ alkenes from a CO and $H_2$ mixture comprising the step of contacting said mixture at a temperature between 150°C and 300°C with a finely-divided substantially silicon-free catalyst composition as described herein comprising non-pyrophoric particles of an iron-carbon component, at least a portion of which is cementite and wherein the particles have average diameters between 1 and 100 nm produced by a laser-driven gas phase pyrolytic decomposition of a volatile compound containing iron and carbon.

The basic catalyst may be prepared by gas phase pyrolytic decomposition of a volatile organic iron-containing compound (optionally in the presence of an additional carbon source) in the presence of a laser emission under conditions of laser power absorption, reactant and/or diluent flow rate and pressure to produce finely divided iron-carbon containing catalyst particles.

The organic-iron-containing compounds generally are iron carbonyls. Compounds such as $Fe(CO)_5$, ferrocene, and iron acetylacetonate are all suitable; $Fe(CO)_5$ is especially preferred. The optional carbon source may act only as a diluent, depending upon reaction conditions, or may add a source of carbon to the pyrolysis reaction. The preferred carbon sources are short chain olefins such as ethylene. Obviously, at least one of the components must absorb the radiated laser energy.

The partial pressure of the organic-iron-containing compounds depends upon the total pressure of the reactor but may be in the range of 20 to 500 torr (2.7 to 66.7 kPa); the partial pressure of the optional carbon source may be from 20 to 500 torr (2.7 to 66.7 kPa) and a diluent such as argon or other noble gas may be included to bring the overall system pressure to a total of 200 to 1000 torr (26.7 to 133.3 kPa).

By "finely divided" Fe-C catalyst particles is meant those having average diameters between 1 and 100 nm, preferably 10—50 nm. The materials usually have a BET surface area of 15 to 50 $m^2$/gm, preferably 20—35 $m^2$/gm. The iron-carbon catalyst is at least a portion e.g. a major portion, of cementite, $Fe_3C$. The catalyst is a mixture of phases and, in addition to the cementite, includes α and γ phase iron. The surface iron of the as-produced catalyst is carbidic. The α and γ-Fe phases appear to be embedded in the cementite. In some cases, the varying phases appear to be more than a simple physical mixture and may constitute a non-equilibrium mixture. A minimum amount of carbonaceous material is present on the exterior surface of the catalyst as a coating. The coating acts as a moderate passivating agent. No hydrogen pretreatment is needed to activate the as-produced base catalyst. The catalyst is not pyrophoric. The catalyst contains less than about 1.0% oxygen and is substantially bereft of silicon. Although the method of producing this catalyst is believed, of itself, to produce a catalyst which is unique, the catalyst desirably contains no more than about 20% total carbon, preferably no more than about 12% total carbon, and most desirably between about 8% and 12% total carbon. Directionally, the higher the percentage of excess matrix carbon, the lower the amount of $C_{10}$ olefins produced.

The catalyst which has been found to be optimum for the preparation of the heavier hydrocarbons contains at least 2% alkali metal or alkaline earth metal, preferably from 2% to 10% by weight.

The laser used is preferably a continuous wave (cw) type capable of producing a flux of about 200 to 10,000 W/$cm^2$ in the reaction zone and further capable of resonant adsorption with a substance in the reaction zone. A $CO_2$ laser of adequate size is desirable. The residence time of the reactants in the laser beam zone should be between 1 and 60 ms. The quench rate for the products leaving the zone should be

such that the total time the reactant/products are at the elevated temperature is 0.15 seconds or less. Quenching may be provided mainly by radiative energy loss from the reaction products.

It is to be understood that the reactor pressures and gas flow rates described herein are not critical to the synthesis of the base catalyst, but are merely convenient for the particular reactor design employed. The only requirements are that the operating conditions be such that the time scale of the reaction be short enough to prevent deposition of excess carbon on the solid particles produced in the reaction, and that temperatures sufficient to drive the reaction be reached. Depending upon the power of the particular laser used to drive the reaction and the design of the particular reactor used to conduct the synthesis, a wide range of reactor pressures and gas flow rates will allow preparation of the catalyst.

By changing the reaction conditions, it is possible to obtain other products from the same reactants. For example, increasing the $Fe(CO)_5$:$C_2H_4$ ratio to 1:4 while maintaining the same laser power yields a product which is substantially all free iron and pyrophoric. Decreasing the residence time of the reactants in the laser beam has substantially the same effect. Similarly, increasing the laser power, or otherwise raising the reaction temperature, increases the carbon content of the product by continued decomposition of $C_2H_4$ after the $Fe(CO)_5$ is depleted. An increase in reaction time would have a similar effect.

The iron-carbon catalyst particles may be used as such to produce olefins; e.g., in an appropriate slurry reactor, or may be supported in one fashion or another as known in the art. The catalyst may be integrated with known supports to produce a larger catalyst matrix which may be handled with more ease.

Promoters such as alkali metals, preferably potassium, or alkaline earth metals, such as magnesium, may be added using known methods. For instance, up to 10% potassium, preferably 2%, may be added to the as-produced Fe-C catalyst by impregnation with an aqueous solution of a potassium salt such as potassium carbonate. More difficulty soluble materials may be ground and mulled with the as-produced Fe-C catalyst prior to a compaction step such as pilling, tabletting or extruding.

Of course, for certain applications the iron carbide catalytic material may be placed on a refractory support such as alumina, silica, mullite, diatomaceous earth, silica-alumina co-mixtures or other materials known to provide high surface area.

The process for conversion of $CO/H_2$ to the various hydrocarbon products using the catalyst discussed above may be a fixed bed or preferably a slurry process. In the slurry process, the catalyst is suspended in a liquid hydrocarbon and the $CO/H_2$ mixture forced through the catalyst slurry allowing good contact between the $CO/H_2$ and the catalyst to initiate and maintain the hydrocarbon synthesis process. The slurry process is described in detail in such articles as Catal. Rev.—Sci. Engr., *21*, 1980, pg. 225 (Kolbol, Rulek).

Advantages of a slurry process over that of a fixed bed process include better control of the exothermic heat produced in the Fischer-Tropsch process during the reaction and better control over catalyst activity maintenance by allowing continuous recycle, recovery, and rejuvenation procedures to be implemented. The slurry process can be operated in a batch or in a continuous cycle, and in the continuous cycle, the entire slurry can be circulated in the system allowing for better control of the primary products residence time in the reaction zone.

The slurry liquid used in the process is a liquid at the reaction temperature, should be chemically inert under the reaction conditions and should be a relatively good solvent for $CO/H_2$ and possess good slurrying and dispersing properties for the finely divided catalyst. Representative classes of organic liquids which can be utilized are high boiling paraffins, aromatic hydrocarbons, ethers, amines, or mixtures thereof. The high boiling paraffins include $C_{10}$—$C_{50}$ linear or branched paraffinic hydrocarbons; the aromatic hydrocarbons include $C_7$—$C_{20}$ single ring and multi- and fused ring aromatic hydrocarbons; the ethers include aromatic ethers and substituted aromatic ethers where the ether oxygen is sterically hindered from being hydrogenated; the amines include long chain amines which can be primary, secondary, and tertiary amines, wherein primary amines preferably contain at least a $C_{12}$ alkyl group in length, secondary amines preferably contain at least two alkyl groups being $C_7$ or greater in length, and tertiary amines preferably contain at least three alkyl groups being $C_6$ or higher in length. Representative examples of specific liquid slurry solvents useful are dodecane, tetradecane, hexadecane, octadecane, cosane, tetracosane octacosane, ditriacontane, hexatritacosane, tetracontane, tetratetracontane, toluene, o-, m-, and p-xylene, mesitylene, $C_1$—$C_{12}$ mono- and multi-alkyl substituted benzenes, dodecylbenzene, naphthalene, anthracene, biphenyl, diphenylether, dodecylamine, di-nonylamine, trioctylamine, and the like. Preferred liquid hydrocarbon slurry solvent is octacosane or hexadecane.

The amount of catalyst used in the liquid hydrocarbon slurry solvent is generally about 10 to 60 g of dry catalyst per 500 g slurry liquid. Preferably about 30 to 50 g dry catalyst per 500 g slurry liquid slurry is utilized, being in about a respective 5:1 to 10:1 weight ratio.

The slurry system, comprising the slurry liquid and finally divided catalyst, is generally stirred to promote good dispersion during the pretreatment in the process to avoid catalyst settling and to eliminate mass transport limitations between the gas and liquid phases.

The operating conditions for this process are generally as found below.

| | | Fixed bed | Slurry |
|---|---|---|---|
| T°C | (heavy hydrocarbons) | 200—250 | 200—250 |
| (pref.) | (heavy hydrocarbons) | 220—240 | 220—240 |
| T°C | (light olefins) | 240—300 | 240—280 |
| (pref.) | (light olefins) | 250—275 | 250—275 |
| Gauge press. (psig) kPa | (heavy hydrocarbons) | (50—500) 345—3448 | (50—500) 345—3448 |
| (pref.) | (heavy hydrocarbons) | (150—500 (1034—3448 | (150—500) 1034—3448 |
| Gauge press. (psig) | (light olefins) | (50—200) 345—1379 | (50—200) 345—1379 |
| (pref.) | (light olefins) | (50—120) 345—827 | (50—120) 345—827 |
| $H_2/CO$ | | 0.5—9:1 | 0.5—9:1 |
| (pref.) | | 1.8—2.5:1 | 1.8—2.5:1 |
| SHSV (volume fresh gas/volume catalyst/hr) | | 100—10,000 | 100—10,000 |
| Stirrer speed (rpm) | | — | 600—4000 |
| Recycle gases | | $C_4^-/CH_4/CO_2$ | $C_4^-/CH_4/CO_2$ |
| Diluent gases | | $N_2/Ar/CH_4/$ light hydro-carbons/$CO_2$ | $N_2/Ar/CH_4/$ light hydro-carbons/$CO_2$ |

A magnetically stabilized fluidized bed as is described in US—A—4,115,927 is also suitable for this reaction.

Having thus described the invention, the following are examples which illustrate the various workings of it. They are not intended to limit the invention in any way.

Example I

The base catalyst was prepared in a high surface area, low excess carbon form by a gas phase pyrolytic decomposition reaction driven by a cw $CO_2$ laser. The reactants were $Fe(CO)_5$ and $C_2H_4$. The $C_2H_4$ also served to absorb energy from the laser beam, allowing rapid heating of the reactants to reaction temperature. Post-reaction quenching is also very rapid, preventing extensive decomposition of the $C_2H_4$ on the catalyst particles and thus minimizing excess carbon content of the solid.

The reactor is shown in Figure 1. It was constructed around a mini-flange 6-way cross. As shown in the Figure, the vertical axis of the apparatus was used for introduction of the reactants and take-off of products. One horizontal axis was used for passage of the laser beam, while the remaining horizontal axis was used for monitoring the reaction. Argon inlets were provided near each of the four windows to prevent deposition of particulates on the windows. The $C_2H_4/Fe(CO)_5$ mixture entered the cell through a tube which was concentric with a slightly larger tube to a point 1—4 mm below the laser beam. The outer tube was used to provide an argon stream surrounding the reactant stream, thereby promoting stable flow of the reactants into the laser beam.

The laser was operated in a cw mode on the 10 P(20) line at 944 $cm^{-1}$. Although not resonant with the 950 $cm^{-1}$ Q-branch of $C_2H_4$, this line is absorbed strongly enough by weaker $C_2H_4$ absorption bands to drive the pyrolytic reaction. The laser produced about 150 W in a beam focused to 6 mm diameter at the reaction zone, yielding a flux of 500 $W/cm^2$.

The synthesis was conducted at a reactor pressure of about 300 torr. The total argon flow to the four cell windows was about 70 standardized $cm^3$/minute ( SCCM) ($cm^3$/min at STP), while the argon flow coaxial to the reactants was also 70 SCCM. The $C_2H_4/Fe(CO)_5$ mixture was provided by bubbling $C_2H_4$ through liquid $Fe(CO)_5$ held at ambient temperature (23°C) where the vapor pressure is 25 torr (3.3 kPa). [Gilbert, A. G.; Sulzmann, K. G. P., *J. Electrochem. Soc.* 1974, 121, 832—834]. The $C_2H_4$ flow rate was about 6 SCCM. Since the $Fe(CO)_5$ will essentially attain its equilibrium vapor pressure in the $C_2H_4$ stream under these flow conditions, the ratio of the reactants in the gas stream is determined by the total reactor pressure; $C_2H_4:Fe(CO)_5=(300—25):25=11:1$.

The laser-driven reaction gave a bright yellow flame, indicating that quite high temperatures were obtained. Under the flow and pressure conditions given above, the residence time of the reactants in the laser beam is 25—40 ms and the quenching rate should be fast enough to keep the total time at high temperature, e.g., above about 500°C, to 0.1 s or less.

The solid products were collected on an 0.5 µm-pore Teflon (Registered Trade Mark) membrane filter. The gaseous products were monitored by gas chromatograph (gc) and infrared detector (ir). The ir showed

that conversion of $Fe(CO)_5$ to products was quantitative under reaction conditions. The characteristic τ (CO) bands of $Fe(CO)_5$ could not be seen in the product gases, though free CO was present. The GC showed that most of the $C_2H_4$ did not react. The gas yields were to some extent dependent upon the linear flow rate of the reactant stream at the laser beam as shown below. Since the reactant stream does undergo some spreading as it enters the reactor, the linear velocity decreases with distance from the inlet tip. Raising the laser beam further above the inlet tip, or alternatively, decreasing the flow rate of the reactants, led to increased residence time of the reactants in the beam. The gas yields then indicated higher reaction temperature, or a longer reaction, or both, as demonstrated by the increase in yields of $C_2H_2$ and $CH_4$ relative to $C_2H_4$.

Measured mole %, TCD

| Gas | High flow | Low flow |
|---|---|---|
| $C_2H_4$ | 64 | 57% |
| CO | 32 | 29% |
| $C_2H_2$ | 3.3 | 12.5% |
| $CO_2$ | 0.67 | 0.08% |
| $CH_4$ | 0.50 | 1.55% |

$H_2$ was also observed, but the peak area is not meaningful (He carrier). A peak for $C_2H_6$ could be observed by eye in the GC trace, but was so weak and broad that the integrator normally did not detect it. The yield was measured at 0.06% of the gases in one instance.

The analysis of one sample of solid prepared by the above method was: Fe, 86.2%; C, 12.74%; O, 1.73%; H, <0.35%. x-ray diffraction showed that the major phase present was $Fe_3C$. The BET surface area was 27 $m^2/g$, and electron spectroscopy (XPS) showed that the surface was carbon rich, with only Fe and C present. The catalyst so prepared was not pyrophoric and did not appear to oxidize significantly in air. Analysis by Mossbauer spectroscopy showed that $Fe_3C$ was the major phase, with smaller amounts of α-Fe and γ-Fe also present.

Example II

Gas streams containing $Fe(CO)_5/C_2H_4$ were pyrolyzed using the method of Example I, with a cw $CO_2$ laser producing about 200 W, to yield powders containing Fe and C. The total pressure of the reactant gases was 385 torr (51.3 kPa). The partial pressure of $Fe(CO)_5$ and the flow rate of the $C_2H_4$ were varied. Analytical results for the powders are shown below.

| Synthesis | (a) | (b) | (c) | (d) |
|---|---|---|---|---|
| $Fe(CO)_5$ partial pressure, (torr) kPa | (92) 12.3 | (73) 9.7 | (73) 9.7 | (30) 3.9 |
| $C_2H_4$ flow rate, $cm^3$/minute | 15 | 15 | 35 | 35 |
| Powder analysis, %Fe | 92.9 | 90.9 | 89.6 | 87.0 |
| Powder analysis, %C | 8.15 | 9.04 | 8.60 | 10.80 |
| Powder surface area, $m^2/g$ | 20.4 | 22.2 | 24.1 | 34.8 |

All powders were shown to be mainly $Fe_3C$ by X-ray diffraction.

These results demonstrate that the powder composition can be controlled by varying the preparation conditions.

Example III

Catalyst (1)

Samples of $Fe_3O_4$ were reduced in flowing $H_2$ at 450°C for 5—7 hours and then treated in $H_2/CO$ at 350°C until the X-ray diffraction pattern indicated that all the iron was converted to a carbide phase, predominantly of the form $Fe_5C_2$ and $Fe_3C$ in a matrix of 40—70% wt. of an amorphous carbon phase. This catalyst was transferred directly to the reactor and brought up to reaction temperature and pressure under a $CO/H_2$ mixture.

Catalyst (2)

A gas stream containing $Fe(CO)_5/C_2H_4$ was pyrolyzed using the method of Example I with a CW $CO_2$

laser to yield a powder containing Fe and C as the only detectable components with ca. 5—15% wt. of an amorphous carbon phase.

The performance of these two catalysts under continuous stirred tank reactor conditions is shown below.

| Catalyst | (1) | (2) |
|---|---|---|
| v/v cat/hr | 2000 | 4000 |
| % CO conversion | 71.9 | 82.5 |
| Wt.% selectivity ($CO_2$ free basis) | | |
| $CH_4$ | 16.1 | 9.5 |
| $C_2°$ | 9.7 | 5.4 |
| $C_2=$ | 3.0 | 7.5 |
| $C_3°$ | 5.6 | 1.3 |
| $C_3=$ | 10.9 | 10.5 |
| $C_4°$ | 3.0 | 1.1 |
| $C_4=$ | 5.0 | 9.0 |
| % olefin in $C_2$—$C_4$ | 50.7 | 77.6 |

Conditions: 270°C, 2/1 $H_2/CO$, 200 SCCM, 75 psi (517 kPa), octacosane solvent, 600 rpm. The $H_2/CO$ mixture was run directly through the reactor without recycle of product gases.

The results demonstrate the high activity and olefin selectivity provided by the laser generated Fe-C catalyst (2) relative to the conventionally prepared iron carbide catalyst (1).

Example IV
Catalyst 3

Samples of the laser generated Fe/C catalyst (2) of the invention were treated with $H_2/CO$ at 350°C to generate an amorphous carbon phase equivalent to that present in the conventionally prepared iron carbide catalyst (1).

The performance of the $H_2/CO$ treated laser generated Fe/C catalyst relative to the conventional iron carbide catalyst (1) is shown below.

| Catalyst | (1) | (3) |
|---|---|---|
| v/v cat/hr | 2000 | 5000 |
| % CO conversion | 71.9 | 33.0 |
| Wt.% selectivity ($CO_2$ free basis) | | |
| $CH_4$ | 16.1 | 11.1 |
| $C_2°$ | 9.7 | 7.3 |
| $C_2=$ | 3.0 | 16.6 |
| $C_3°$ | 5.6 | 1.0 |
| $C_3=$ | 10.9 | 9.0 |
| $C_4°$ | 3.0 | 0.6 |
| $C_4=$ | 5.0 | 6.6 |
| % olefin in $C_2$—$C_4$ | 50.7 | 80.0 |
| % olefin in $C_{10}+$ | N.A. | 35.0 |

Conditions: 270°C, 2/1 $H_2/CO$, 200 SCCM, 75 psi (517 kPa), octacosane solvent, 600 rpm.

These results show the improved olefin selectivity achieved with the laser generated Fe/C catalyst (3) that contains amorphous carbon at levels found in the conventional iron carbide synthesis catalyst (1). However, the presence of excess matrix carbon decreases (as shown by comparison with catalyst (2)) the amount of liquid $C_{10}^+$ olefin.

Example V

Catalyst (4)

A laser synthesized Fe/Si/C composition analogous to that described by Gupta and Yardley was prepared by CW $CO_2$ laser pyrolysis $Fe(CO)_5/C_2H_4/SiH_2(CH_3)_2$.

The behavior of this material relative to the Si-free catalyst (2) of the invention are shown below.

| Catalyst | (2) | (4) |
|---|---|---|
| v/v cat/hr | 4000 | 500 |
| % CO conversion | 82.5 | 5.0 |
| Wt.% selectivity ($CO_2$ free basis) | | |
| $CH_4$ | 9.5 | 15.9 |
| $C_2°$ | 5.4 | 6.0 |
| $C_2=$ | 7.5 | 3.0 |
| $C_3°$ | 1.3 | 15.0 |
| $C_3=$ | 10.5 | 12.0 |
| $C_4°$ | 1.1 | 6.0 |
| $C_4=$ | 9.0 | 4.0 |
| % olefin in $C_2$—$C_4$ | 72.6 | 41.0 |

Conditions, 270°C, 2/1 $H_2/CO$, 200 SCCM, 75 psi (517 kPa), octacosane solvent, 600 rpm.

These results demonstrate the superior activity and selectivity provided by the Fe/C catalyst (2) of this invention relative to the Fe/Si/C catalyst (4).

Example VI

The conventionally prepared iron carbide catalyst (1), the laser generated Fe/C (2) and Fe/Si/C (4) catalysts were examined under CSTR conditions: 270°C, 2/1 $H_2/CO$, 200 standardized $cm^3$/min (SCCM), 75 psig (517 kPa gauge), octacosane solvent, 600 rpm. The yield and composition of liquid products collected at 4°C and 15 psig (103 kPa gauge) were determined after 48 hours and are shown below.

| Catalyst | 1 | 2 | 4 |
|---|---|---|---|
| Wt.% $C_{10}$ ($CO_2$ free basis) | nil | 1.9 | nil |
| Distribution (%) | | | |
| 1-olefin | nil | 47.2 | nil |
| n-paraffin | — | 10.4 | — |
| n-alcohol | — | 1.1 | — |
| others | — | 36.5 | — |

The laser generated Fe/C catalyst (2) of this invention in contrast to the iron carbide (1) or laser generated Fe/Si/C catalyst (4) generates a recoverable $C_{10}$ fraction containing high levels of 1-olefin.

Example VII

The laser generated Fe/C catalyst (2) was examined at 100 psig (689.5 kPa gauge) at 270°C and 240°C under continuously stirred tank reactor (CSTR) conditions with 2/1 $H_2/CO$. The results of those tests are shown below:

| | | |
|---|---|---|
| Temp. (°C) | 240 | 270 |
| v/v cat/hr | 4000 | 4000 |
| % CO conversion | 43.9 | 89.3 |
| Wt.% selectivity ($CO_2$ free basis) | | |
| $CH_4$ | 5.9 | 6.4 |
| $C_2°$ | 0.01 | 2.0 |
| $C_2=$ | 6.0 | 4.9 |
| $C_3°$ | 4.2 | 3.3 |
| $C_3=$ | 8.1 | 9.7 |
| $C_4°$ | 0.8 | 0.8 |
| $C_4=$ | 5.6 | 8.3 |
| % olefin in $C_2$—$C_4$ | 80 | 79 |

These results demonstrate the high activity and olefin selectivity provided by the catalyst of our invention over a reasonably wide range of operating temperatures.

Example VIII

The laser generated Fe/C catalyst (2) of this invention was impregnated with $K_2CO_3$ to yield a material containing 2% wt. K. This material was examined at 270°C, 2/1 $H_2$/CO, 4000 V/V cat/hr, 75 psig (517 kPa gauge), octacosane, 600 rpm. The results are shown below:

| | |
|---|---|
| % CO conversion | 66.5 |
| Wt.% selectivity ($CO_2$ free basis) | |
| $CH_4$ | 5.6 |
| $C_2°$ | nil |
| $C_2=$ | 3.7 |
| $C_3°$ | 0.5 |
| $C_3=$ | 4.3 |
| $C_4°$ | 0.3 |
| $C_4=$ | 3.5 |
| % olefin in $C_2$—$C_4$ | 93.5 |

The results demonstrate the high olefin selectivity provided by the alkali promoted catalyst of our invention.

Example IX

A laser generated Fe/C catalyst made according to Example I with about 5—15% wt. of amorphous carbon was impregnated with $K_2CO_3$ to yield a material containing about 2% wt. K. This material was examined in a continuously stirred tank reactor at reaction conditions of 270°C, 2/1 $H_2$/CO, 4000 V/V cat/hr, 75 psig (517 kPa gauge), octacosane, 600 rpm. The results are shown below:

| | |
|---|---|
| % CO conversion | 66.5 |
| Wt.% selectivity ($CO_2$ free basis) | |
| $CH_4$ | 5.6 |
| $C_2°$ | nil |
| $C_2=$ | 3.7 |
| $C_3°$ | 0.5 |
| $C_3=$ | 4.3 |
| $C_4°$ | 0.3 |
| $C_4=$ | 3.5 |
| $C_5^+$ | 82.1 |
| % olefin in $C_2$—$C_4$ | 93.5 |

The results demonstrate the high olefin selectivity provided by the alkali promoted catalyst of our invention.

Example X

A mixture of 2.0 g of the laser generated Fe/C powder and 6 g of MgO were pelletized, crushed, and sieved (80—150 Tyler mesh, 0.177 to 0.105 mm) and examined in a down flow fixed-bed reactor at 221°C, 2/1 $H_2$/CO, 3000 V/V/hr, and 75 psig (517 kPa gauge).

| | |
|---|---|
| % CO conversion | 45.7 |
| to $CO_2$ | 9.0 |
| to hydrocarbons | 36.7 |
| Wt.% selectivity ($CO_2$ free basis) | |
| $CH_4$ | 5.0 |
| $C_2$—$C_4$ | 9.0 |
| $C_5^+$ | 86.0 |
| $C_2=/C_2°$ | 0.7 |
| $C_3=/C_3°$ | 4.0 |

The results demonstrate the usefulness of the laser generated catalyst in the synthesis of $C_5$+ hydrocarbons.

Example XI

A 300 cm³ Parr Continuously Stirred Tank Reactor (CSTR) was charged with 8.0 g of a conventionally prepared catalyst $Fe_{4.75}Co_{.25}C_2$/x g atom % K where x=0, 2 and 10. The material contains an additional carbon phase, ca. 50—70% wt. The reactor was attentively charged with 2.0 g of a laser generated carbide catalyst $Fe_3C_y$/x g atom % K where y is from 1 to 2 and x=0 or 2.0. A slurry medium consisting of 70 g of octacosane $C_{28}H_{58}$ containing trace levels of sodium bromide, ≤300 ppm, was also charged, the system purged with a gas mixture $H_2$:CO:$N_2$, 60:30:10 molar ratio and then brought to reaction conditions: 240°C, 75 psi (517 kPa), 60:30:10 standardized cm³/minute (SCCM) $H_2$:CO:$N_2$, with stirring at 600 rpm. An exit gas analyzer was employed to determine the extent of CO hydrogenation, the carbon efficiency to $CH_4$ and the olefin content of the $C_2$—$C_4$ fraction. Higher molecular weight products were analyzed off-line on completion of the experiment. Results are provided in the Table below. The high volumetric activity and good olefin selectivity of the laser generated catalyst, even with 0% K are clearly shown.

The laser generated catalyst with about 2% K was found to provide unusually high selectivity for production of hydrocarbon wax even when compared to the Fe-Co analog with 10% K.

TABLE

| | FeCoC cat. | | | | FeC cat. | | | |
|---|---|---|---|---|---|---|---|---|
| %K | % conv | % olefin in $C_2$—$C_4$ | %$CH_4$ | —$C_5$+ | % conv | % olefin in $C_2$—$C_4$ | %$CH_4$ | $C_5$+ |
| 0 | 72 | 37 | 20 | 40 | 55 | 86 | 7 | 78 |
| 2 | 48 | 80 | 10 | 64 | 38 | 87 | 5 | 88 |
| 10 | 40 | 87 | 10 | 64 | — | — | — | — |

**Claims**

1. A catalyst composition comprising finely divided non-pyrophoric particles containing iron and carbon components, at least a portion of which is cementite, produced in a reaction zone from a volatile compound containing iron and carbon in the presence of laser radiation under such conditions of laser flux density, power absorption, a volatile iron and carbon compound concentration and pressure sufficient to produce said particles, the particles having average diameters between 1 and 100 nm, and the composition being substantially free of silicon.

2. A composition according to claim 1 wherein at least a portion of the iron is in the α and γ phase.

3. A composition according to claim 1 or claim 2 wherein the particles contain at least some free carbon.

4. A composition according to any one of claims 1 to 3 comprising at least one promoter, for example an alkali or alkaline earth metal, in an amount of between 2 and 10% by weight based on the catalyst composition.

5. A composition according to claim 4 wherein said promoter is potassium or magnesium.

6. A composition according to any one of claims 1 to 5 wherein the particles are supported on a matrix.

7. A process for producing $C_5$ hydrocarbons from a CO and $H_2$ mixture comprising the step of contacting said mixture at a temperature between 200°C and 250°C with a substantially silicon-free catalyst composition according to any one of claims 1 to 6 comprising non-pyrophoric particles of an iron-carbon component, at least a portion of which is cementite and wherein the particles have average diameters between 1 and 100 nm produced by a laser-driven gas phase pyrolytic decomposition of a volatile compound containing iron and carbon, and a promoter selected from alkali metals and alkaline earth metals and preferably present in an amount of from 2 to 10 weight percent based on the catalyst composition.

8. A process according to claim 7 wherein said temperature is between 220°C and 240°C.

9. A process for producing $C_2$—$C_{15}$ alkenes from a CO and $H_2$ mixture comprising the step of contacting said mixture at a temperature between 150°C and 300°C with a finely-divided substantially silicon-free catalyst composition according to any one of claims 1 to 6 comprising non-pyrophoric particles of an iron-carbon component, at least a portion of which is cementite and wherein the particles have average diameters between 1 and 100 nm produced by a laser-driven gas phase pyrolytic decomposition of a volatile compound containing iron and carbon.

10. A process according to claim 9 wherein said temperature is between 220° and 280°C.

11. A process according to claim 9 or claim 10 wherein the catalyst composition additionally contains at least one promoter selected from alkali and alkaline earth metals.

12. A process according to claim 11 wherein the promoter is potassium or magnesium.

13. A process according to any one of claims 9 to 12 wherein the catalyst is in bulk form or in a slurry.

14. A process according to any one of claims 7 to 13 wherein the ratio of $H_2$:CO is between 1:3 and 5:1.

**Patentansprüche**

1. Katalysatorzusammensetzung, die feinteilige nicht-pyrophore Teilchen umfaßt, die Eisen und Kohlenstoffbestandteile enthalten, von denen mindestens ein Teil Zementit ist, und die in einer Reaktionszone aus einer flüchtigen, Eisen und Kohlenstoff enthaltenden Verbinding un Gegenwart von Laserstrahlung unter solchen Bedingungen von Laserstrahlungsdichte, Energieabsorption, Konzentration an flüchtiger, Eisen und Kohlenstoff enthaltender Verbindung und Druck hergestellt sind, die ausreichen, die Teilchen herzustellen, wobei die Teilchen einen durchschnittlichen Durchmesser zwischen 1 und 100 nm besitzen und die Zusammensetzung im wesentlichen frei von Silicium ist.

2. Zusammensetzung nach Anspruch 1, bei der mindestens ein Teil des Eisens in der α- und γ-Phase ist.

3. Zusammensetzung nach Anspruch 1 oder 2, bei der die Teilchen mindestens etwas freien Kohlenstoff enthalten.

4. Zusammensetzung nach einem der Ansprüche 1 bis 3, die mindestens einen Promotor,

beispielsweise ein Alkali- oder Erdalkalimetall, in einer Menge von 2 bis 10 Gew.% bezogen auf die Katalysatorzusammensetzung enthält.

5. Zusammensetzung nach Anspruch 4, in der der Promotor Kalium oder Magnesium ist.

6. Zusammensetzung nach einem der Ansprüche 1 bis 5, in der die Teilchen auf eine Matrix aufgebracht sind.

7. Verfahren zur Herstellung von $C_5$-Kohlenwasserstoffen aus einer CO und $H_2$ Mischung, bei dem die Mischung bei einer Temperatur zwischen 200°C und 250°C mit einer im wesentlichen siliciumfreien Katalysatorzusammensetzung nach einem der Ansprüche 1 bis 6 kontaktiert wird, die nicht-pyrophore Teilchen einer Eisen - Kohlenstoff - Komponente, von der mindestens ein Teil Zementit ist, wobei die Teilchen einen durchschnittlichen Durchmesser von 1 bis 100 nm besitzen und durch eine laserbetriebene pyrolytische Gasphasenzersetzung einer flüchtigen, Eisen und Kohlenstoff enthaltenden Verbindung hergestellt sind, und einen Promotor ausgewählt aus Alkalimetallen und Erdalkalimetallen, der vorzugsweise in einer Menge von 2 bis 10 Gew.% bezogen auf die Katalysatorzusammensetzung vorhanden ist, umfaßt.

8. Verfahren nach Anspruch 7, bei dem die Temperatur zwischen 220°C und 240°C liegt.

9. Verfahren zur Herstellung von $C_2$—$C_{15}$ Alkenen aus eine CO und $H_2$ Mischung, bei dem die Mischung bei einer Temperatur zwischen 150°C und 300°C mit einer feinteiligen, im wesentlichen siliciumfreien Katalysatorzusammensetzung nach einem der Ansprüche 1 bis 6 kontaktiert wird, die nicht-pyrophore Teilchen einer Eisen - Kohlenstoff - Komponente umfaßt, von der mindestens ein Teil Zementit ist, wobei die Teilchen einen durchschnittlichen Durchmesser zwischen 1 und 100 nm besitzen und durch eine lasergetriebene pyrolytische Gasphasenzersetzung einer Eisen und Kohlenstoff enthaltenden flüchtigen Verbindung hergestellt sind.

10. Verfahren nach Anspruch 9, bei dem die Temperatur zwischen 220°C und 280° liegt.

11. Verfahren nach Anspruch 9 oder 10, bei dem die Katalysatorzusammensetzung zusätzlich mindestens einen Promotor ausgewählt aus Alkali- und Erdalkalimetallen enthält.

12. Verfahren nach Anspruch 11, bei dem der Promotor Kalium oder Magnesium ist.

13. Verfahren nach einem der Ansprüche 9 bis 12, bei der Katalysator als Masse oder in einer Aufschlämmung vorliegt.

14. Verfahren nach einem der Ansprüche 7 bis 13, bei dem das Verhältnis $H_2$:CO zwischen 1:3 und 5:1 liegt.

**Revendications**

1. Une composition catalytique comportant des particules non-pyrophoriques, finement divisées, contenant des constituants du fer et du carbone, dont au moins une partie est de la cémentite, obtenue dans une zone de réaction à partir d'un composé volatil renfermant du fer et du carbone en présence d'une radiation laser sous des conditions telles de densité de flux du laser, d'absorption d'énergie, de concentration et de pression d'un composé volatil du fer et du carbone qu'elles produisent lesdites particules, les particules présentant des diamètres moyens entre 1 et 100 nm, et la composition étant pratiquement exempte de silicium.

2. Une composition selon la revendication 1, dans laquelle au moins une partie du fer est dans les phases α et γ.

3. Une composition selon la revendication 1 ou la revendication 2, dans laquelle les particules contiennent au moins quelques carbones libres.

4. Une composition selon l'une quelconque des revendications 1 à 3, comportant au moins un promoteur, par exemple un métal alcalin ou alcalino-terreux, en une quantité entre 2 et 10% en poids, en se basant sur la composition catalytique.

5. Une composition selon la revendication 4, dans laquelle ledit promoteur est du potassium ou du magnésium.

6. Une composition selon l'une quelconque des revendications 1 à 5, dans laquelle les particules sont supportées sur une matrice.

7. Un procédé pour la production d'hydrocarbures en $C_5$ à partir d'un mélange de CO et de $H_2$, comportant l'étape de mettre le mélange en contact, à une température entre 200°C et 250°C, avec une composition catalytique pratiquement exempte de silicium selon l'une quelconque des revendications 1 à 6, comportant des particules non-pyrophoriques d'un constituant fer-carbone, dont au moins une partie est de la cémentite et dans laquelle les particules présentant des diamètres moyens entre 1 et 100 nm, obtenues par une décomposition pyrolytique activée par laser en phase gazeuse, d'un composé volatil renfermant du fer et du carbone, et un promoteur choisi parmi les métaux alcalins et les métaux alcalino-terreux et, de préférence, présent en une quantité de 2 à 10 pourcents en poids, en se basant sur la composition catalytique.

8. Un procédé selon la revendication 7, dans laquelle ladite température est entre 220°C et 240°C.

9. Un procédé pour la production d'alcènes en $C_2$ à $C_{15}$ à partir d'un mélange de CO et de $H_2$, comportant l'étape de mettre en contact de ce mélange à une température entre 150°C et 300°C, avec une composition catalytique finement divisée, pratiquement exempte de silicium, selon l'une quelconque des revendications 1 à 6, comportant des particules non-pyrophoriques d'un constituant fer-carbone, dont au

moins une partie est de la cémentite et dans lequel les particules présentent des diamètres moyens entre 1 et 100 nm, obtenues par une décomposition pyrolitique activée par laser en phase gazeuse d'un composé volatile renfermant du fer et du carbone.

10. Un procédé selon la revendication 9, dans lequel ladite température est entre 220°C et 280°C.

11. Un procédé selon la revendication 9 ou la revendication 10, dans lequel la composition catalytique comprend en outre au moins un promoteur choisi parmi les métaux alcalins et alcalino-terreux.

12. Un procédé selon la revendication 11, dans lequel le promoteur est du potassium ou du magnésium.

13. Un procédé selon l'une quelconque des revendications 9 à 12, dans lequel le catalyseur est un vrac ou en suspension épaisse.

14. Un procédé selon l'une quelconque des revendications 7 à 13, dans lequel le rapport $H_2$:CO est entre 1:3 et 5:1.

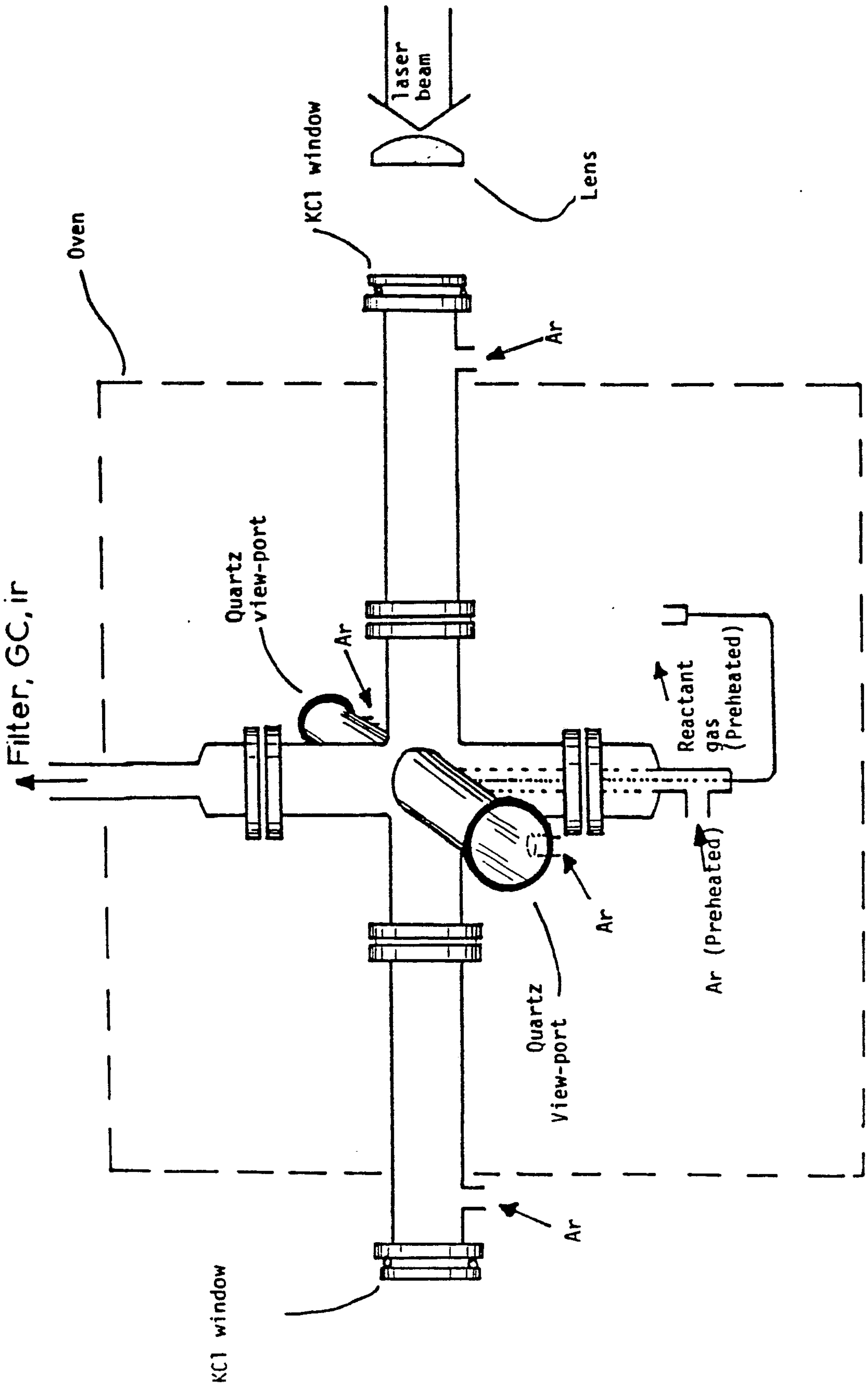


Figure 1. Laser Synthesis Reactor